# EUROPEAN PATENT APPLICATION

(11) **EP 4 517 019 A1**
(43) Date of publication of application: **05.03.2025**
(21) Application number: 23194713.6
(22) Date of filing: 31.08.2023
(51) Int. Cl.: E04B 2/42, C12N 1/14, E04B 1/12, E04C 1/40, E04B 2/02

(54) **CONSTRUCTION ELEMENT AND METHOD OF BUILDING A CONSTRUCTION COMPONENT**

(71) Applicant: Comfortdak B.V., 3707 TB Zeist (NL)
(72) Inventor: BRANDERHORST, Rudolf Hendrik, 5037BA Tilburg (NL)
(74) Representative: De Vries & Metman

(57) **Abstract**

The invention relates to a construction element, preferably a load-bearing construction element (1), for building a construction component, such as a wall (22, 30) or roof, and comprising a block (2) having opposite surfaces and one or more through-holes (3) extending from one surface to the other. The block (2) is a molded block and at least one of the trough-holes (3) slidably holds a positioning and load-bearing peg (5).

## Description

The invention relates to a construction element, preferably a load-bearing construction element, for building a construction component, such as an external or internal wall or a roof, and comprising a block having opposite and preferably parallel surfaces, in particular upper and lower surfaces, and one or more through-holes extending from one surface to the other.

US 2016/244368 relates to structural blocks used in the construction of buildings and civil engineering structures. The blocks can comprise hemp hurd and fibers, flax fiber, hydraulic lime and hydrated lime. In one aspect, the blocks may comprise a body shape configured so as to allow it to interlock with other blocks in the construction of a structure. In an aspect, "structural blocks are provided that may be configured to interlock with complimentary blocks in the construction of a structure. In one embodiment, the structural block may accommodate an embedded member or strut protruding from the surface of one side of the block and a recess on another side."

It is an object of the present invention to provide an improved construction element that facilitates building walls and the like.

To this end, in the construction element according to the present invention the block is a molded block and at least one of the through-holes slidably holds a positioning and load-bearing peg.

In an embodiment, the block comprises a plurality of, preferably at least three, preferably at least four, e.g. eight, through-holes extending from one surface to the other and at least two of the holes, preferably all of the holes, slidably hold a positioning and load-bearing peg.

There are various ways of holding the pegs in the through-holes, which include (mild) adhesives or break elements, that hold the pegs in the through-holes and release the pegs when a block containing the pegs is placed on top of the pegs extending from a lower, already installed layer of blocks, and are forced e.g. pushed out by the lower pegs. In an embodiment, the peg or pegs is/are clampingly held inside the through-holes, preferably by resilience and/or compression of the block, in particular at the walls of the through-holes. The pegs are made from a load-bearing material, such as wood. It is preferred that the pegs are elongated, e.g. have a length that is at least twice, preferably at least trice the widths of the peg.

The construction element according to the present invention facilitates accelerated building of e.g. a wall or a house, in particular by automatic extension of the positioning and load-bearing pegs in each newly laid layer, as will be explained in detail below. Further, the construction element according to the present invention combines internal positioning features, load-bearing capability, and/or lateral stability of a wall or the like with design freedom in including specific functions, such as insulation, and/or features, such as tongue and groove, conduits, and/or attachment points, such as battens, in the molded block.

In embodiments, the block comprises tongues and grooves, e.g. to improve sealing between stacked blocks and thus further enhance insulation, and/or comprises further through-holes or channels, e.g. extending from bottom to top and/or from side to side, for conduits, electrical wires, and/or for tension anchors and/or one or more, preferably most, preferably all of the external edges of the block are chamfered, e.g. to facilitate removing the blocks form a mold.

In embodiments, the through-holes and the pegs are cylindrical, e.g. heaving a square, polygonal, or round cross-section, and/or the block is a parallelepiped, i.e. a three-dimensional body formed by six parallelograms, in particular a rectangular cuboid, i.e. has six at least substantially rectangular faces. In an embodiment, said surfaces, in particular the top and bottom surfaces, have an aspect ratio of 1:N, where N is an integer, e.g. 1, 2, 3 or 4. It is preferred that the blocks are at least 50 centimeters (cm) long (in the horizontal direction, after installation) at least 20 cm wide, and at least 20 cm deep.

In an embodiment, the pattern of the through-holes, when seen in top view, comprises at least two groups of through-holes and at least one of the groups is rotationally symmetrical, preferably of the order 4.

A pattern is rotationally symmetrical if, when the pattern is rotated on its own axis, the shape of the object looks the same. The order of the symmetry describes how many times the object will appear the same while rotating through 360°. E.g. if it matches twice, it is order 2. Rotational symmetry facilitates e.g. including corners in a wall with staggered layers.

To enhance stability of the block, in an embodiment, the distance between the side walls of the block and the pegs is at least 5 cm preferably at least 7 cm.

Suitable materials for the block include lime concrete, gypsum, hemp, glass wool, and rockwool. In an embodiment, the block is made from mycelium, preferably from at least one fungus selected from the group consisting of Pleurotus ostreatus, Pleurotus eryngii, Stropharia Rugosoannulata, Trametes versicolor, Ganoderma Lucidum, Phanerochaete chrysosporium, Bjerkandera adusta, Lentinula edodes, Pycnoporus cinnabarinus, Pycnoporus sanguineus, Grifola frondosa, Schizophyllum commune, Neolentinus lepideus, and Heterobasidiom annosum.

Mycelium provides excellent insulation. Also, if the blocks are made from an organic material, such as mycelium, and the pegs are made from an organic material, such as wood, or a recyclable material, the construction elements enable completely circular building of construction components, such as the walls of a house.

Suitable substrates for creating mycelium composite include wood materials, e.g. particles, such as saw dust and wood shavings, and materials from grain, maize, rice, or hemp.

Materials that can be added, e.g. up to a total amount of 40 wt% of the substrate in total, to the substrate include vegetable materials such as cucumber, peppers, grass, reed, beer broth, potato steam peel, root pulp and used growing substrates from greenhouses. Other examples are polystyrene, plastics, and cardboard materials, as well as inorganic materials such as perlite and vermiculite, preferably to obtain a substrate with a low carbon footprint to replace materials with a high carbon footprint.

Another embodiment comprises the inclusion of reinforcements, such as reinforcing fibers, e.g. filaments of staple fibers, or rods or beams in the mixture and/or the inclusion of chunks of mycelium composite in the mixture. The latter option facilitates the production of larger blocks, e.g. blocks having a height equal to or larger than 30 centimeters, a width equal to or larger than 30 centimeters and a length equal to or larger than 60 centimeters.

The fungus digests the nutrient components in the substrate over a period of time sufficient to grow hyphae and to allow the hyphae to form a network of interconnected mycelia cells through and/or around the discrete particles and/or the chunks in the substrate thereby bonding the discrete particles and/or chunks together to form a (further) mycelium composite and bonding the composite to the walls of the enclosure.

The chunks can be prepared by shredding a mycelium composite. In an embodiment, the chunks are dried, e.g. by means of heat and/or vacuum e.g. in a drying chamber.

In an embodiment, the chunks have an average diameter in a range from 0,5 to 10 centimeters, preferably 1 to 8 centimeters, preferably 2 to 7 centimeters. In a refinement, at least 80 wt%, preferably at least 90 wt% of the chunks have a diameter in that range.

The at least one fungus is preferably a white rot fungus and preferably one that grows relatively quickly and/or is able to accept materials that are strange to its habitat.

Suitable nutrients include sugar, oatflakes, flour, rejected food, and human and animal hair.

In a further aspect, the growth of the fungus is stopped, in particular inactivated or killed, by means of heating, reduced pressure (vacuum), freezing, radiation and/or drying.

In an embodiment, additional elements, in particular elements on the outer surface of the construction element, such as battens providing fixing points for, in particular a façade or wall cladding, are integrated in the mycelium, preferably with the network of hyphae having grown through these additional elements to strength the bond.

The invention also relates to a kit for building a construction component, comprising a plurality of construction elements according to any one of the preceding claims, and a set of separate (initiator) pegs and preferably a base for the construction component.

In an embodiment, the kit comprises empty blocks, i.e. blocks without pegs.

In an embodiment, the separate pegs have at least two, preferably at least three different lengths.

The invention also relates to a method of building a construction component, such as a wall, roof or floor, comprising the steps of positioning a first layer of blocks on a base and positioning pegs in a pattern on the base, such that the pegs extend from the top surfaces of the blocks, and placing a second and preferably further layers of construction elements as described above on the first layer and, if present, subsequent layers, such that the pegs in a previous layer push the pegs in a subsequent layer of construction elements up and out.

US 2021/189723 relates to a construction element includes a frame and a block that slides in the frame when subjected to an external force. The block forms a tenon between two superimposed frames that form mortises. Construction elements fixed together laterally form modules that can be assembled in offset from one level to another to form a wall. The frame is preferably an object formed of a plurality of flat panels parallel to the first direction, so as to delimit the cavity in any direction perpendicular to the first direction. For example, each panel can be formed of a plate. The frame is preferably devoid of grooves on its outer surface.

US 2010/0162650 relates to means for and a method of erecting block drywall wherein the blocks, or bricks, are, for the most part, rectangular in appearance and each provided, on their undersides, with a longitudinal and centrally oriented cavity extending the full length of each block. An additional feature may be the use of short pin members, for instance in the form of tubes, which are dimensioned to fit snugly into the holes in the blocks.

US 2004/0226246 discloses a self interlocking block system wherein blocks with one or more apertures are interconnected by inserting a hollow peg through the aligned apertures of two vertically adjacent blocks such that a portion of the hollow peg extends into the aperture of the top block and a portion of the hollow peg extends into the aperture of the bottom block, resulting in a vertical shaft extending from the top row of blocks through the bottom row of blocks to the base surface. The vertical shafts may be filled with one or more vertical support bars and/or filler material to provide additional support.

ES 2 497 415 relates to a "Procedure for the growth of organic and biodegradable structures from agricultural waste and mushroom mycelium, and its use as insulating components in construction, characterized by its design and manufacture of coherent thermal insulation structures with certain rigidity, 100% organic and biodegradable, using for this agricultural waste (straw, wood shavings, leaves, seed husks ...) and seeds of different species of fungus (pleurotus ostreatus, lentinula edodes, ganoderma lucidum ...)."

WO 2008/073489 relates to a composite material that is comprised of a substrate of discrete particles and a network of interconnected mycelia cells bonding the discrete particles together. The composite material is made by inoculating a substrate of discrete particles and a nutrient material with a preselected fungus. The fungus digests the nutrient material over a period of time sufficient to grow hyphae and to allow the hyphae to form a network of interconnected mycelia cells through and around the discrete particles thereby bonding the discrete particles together to form a self-supporting composite material.

Figure 16 of WO 2008/073489 shows a composite wall panel with molded features, such as a conduit (26), an electrical outlet (27) and wires (28), that are embedded in the composite material of the panel and have an end in communication with an exterior surface of the composite material. These elements are included within the panel during the growth processes in such a manner that they become part of the final monolithic composition.

The invention will now be explained in more detail with reference to the figures, which schematically show an embodiment according to the present invention.

Figures 1A and 1B are a perspective view and a top plan view of a first example of a construction element according to the present invention.

Figure 2 is a perspective view of a second example of a construction element according to the present invention, provided with tongues and grooves, with battens, and with chamfered edges.

Figures 3A to 3E show steps of a method for building a wall and corner in accordance with the present invention.

Figure 4 shows a wall and corner on a screw pile foundation.

Figure 5 shows a T-shaped wall, e.g. an external wall and an integrated internal wall.

Figures 6 and 7 show a wall reinforced with tension anchors.

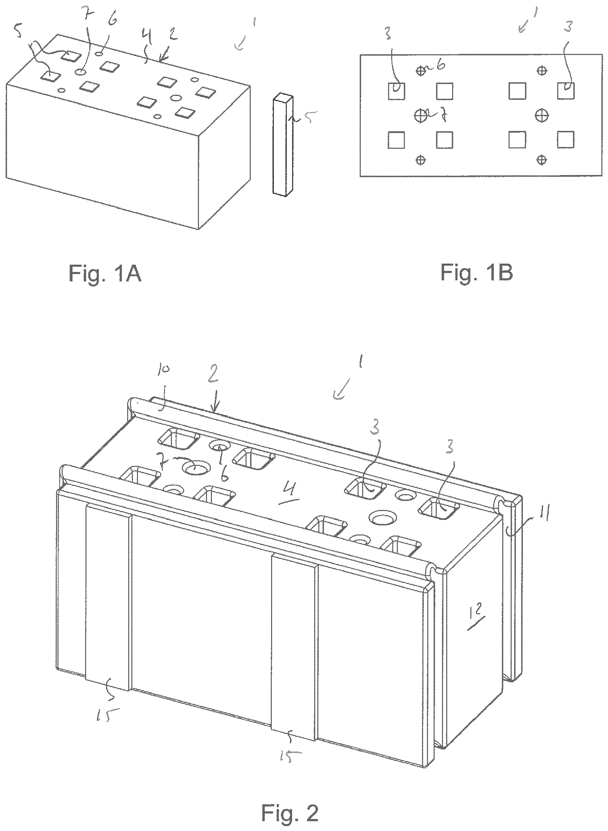

Figures 1A and 1B show a first example of a construction element 1 according to the present invention, for building e.g. the external and/or internal walls of a house. The construction element comprises a rectangular block 2, e.g. 30 x 30 x 60 centimeters in size and of a slightly compressible material, which material will be discussed in detail below. The block comprises a plurality of, in this example eight, through-holes 3 extending from the bottom surface to the top surface 4 of the block ('top' and 'bottom' referring to the orientation of the element during its intended use). Each of the through-holes is cylindrical, with a square cross-section, and a positioning and load-bearing peg 5 is slidably clamped inside each of the through-holes.

The pegs 5 are also cylindrical and have a square cross-section, e.g. 4 x 4 centimeter, that is slightly larger, e.g. 1% larger, than the cross-section of the through-holes, such that when the pegs are be pressed into the through-holes, the pegs cause the through-holes to slightly expand and thus provide a clamping force, which holds the pegs in the through-holes unless pressed out, as will be explained below.

The through-holes, when seen in top view (Figure 1B), are arranged in a pattern comprising two groups of four through-holes each. The pegs in each group are arranged in a square, i.e. each group is rotationally symmetrical of the order 4.

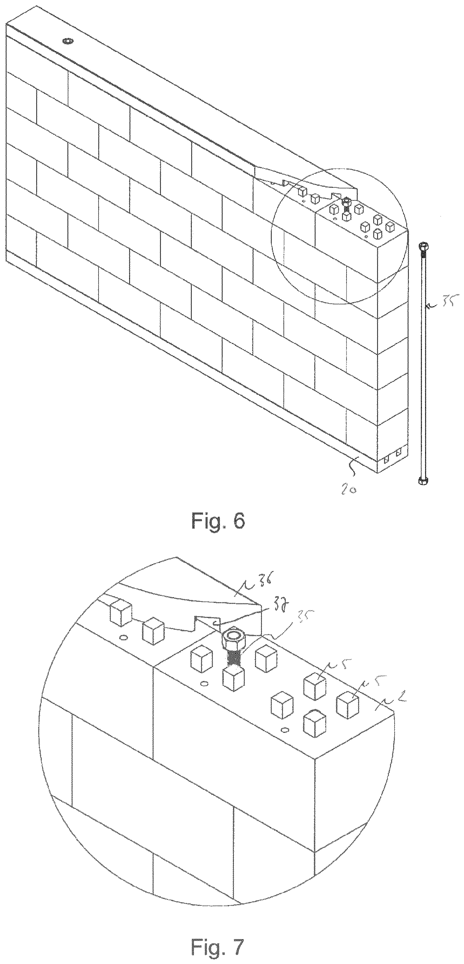

The block comprises additional through-holes 6 for conduits and/or electrical wires, and/or additional through-holes 7 for tension anchors (shown in Figures 6 and 7).

Figure 2 shows a second example of a construction element according to the present invention, wherein the block comprises tongues 10 and grooves 11, e.g. tongues on its top surface 3 and on one of its mating side surfaces 12 and grooves on its lower surface and on its remaining mating side surface, e.g. to further enhance positioning, stability of the finished construction component and/or sealing between stacked blocks and thus further enhancing insulation. The construction element also comprises battens providing fixing points for, in particular, a façade or wall cladding.

All of the external edges of the block are chamfered, including the edges surrounding the openings of the further through-holes 6, 7 or channels for conduits, electrical wires, and/or for tension anchors, as well as all of the external edges of the block are chamfered, e.g. to facilitate removing the blocks form a mould.

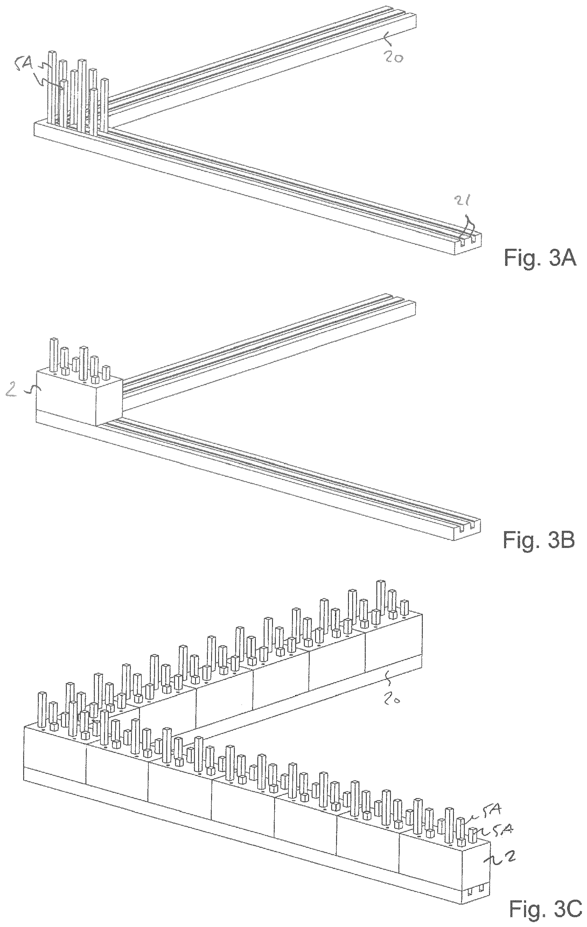

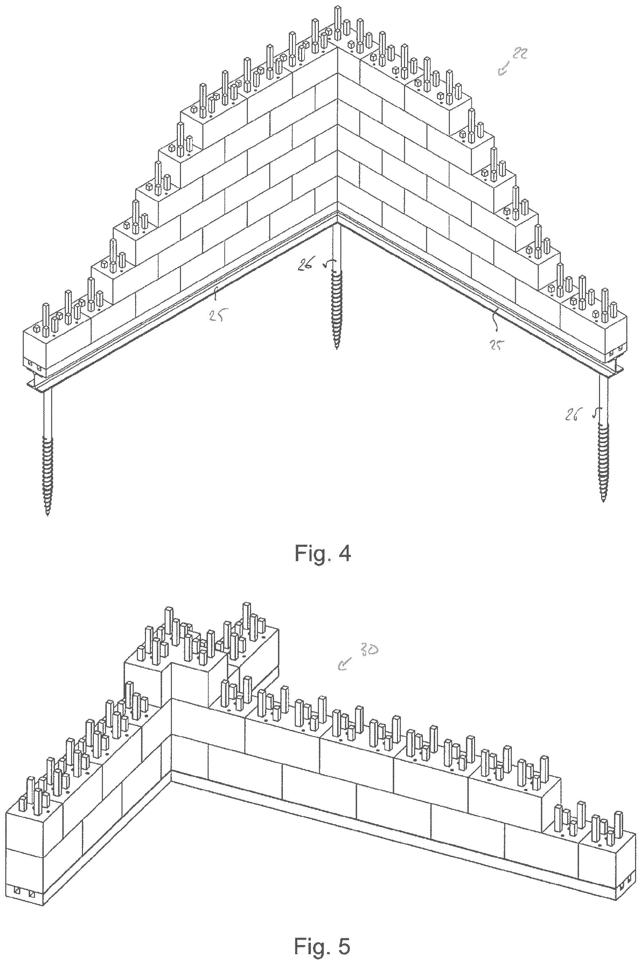

Figures 3A to 3E show steps of a method for building a wall and corner with the blocks shown in Figures 1 and 2. In a first step, shown in Figure 3A, a base plate, e.g. a beam 20 having two grooves 21 running the length of the beam, is placed on a foundation (an example of a foundation is discussed with reference to Figure 4 below). A first series of pegs 5A are placed in the grooves in patterns corresponding to the patterns of the through-holes in the blocks. Next, as shown in Figure 3B, a block is placed over the pegs. As a matter of course, it is also possible to first place the block(s) 2 on the base plate 20 and then insert the first series of pegs 5a in the trough-holes 3 in the blocks 2 and in the grooves 21 in the beams.

The pegs in the first series have different lengths, e.g. the length of each peg is the sum of the depth of the grooves plus the height of the blocks plus a variable part resulting in four different lengths, preferably four different lengths in each group of four. The different length provide more homogenous resistance against e.g. lateral loads.

The first layer of blocks and pegs, shown in Figure 3C, is completed in the same way.

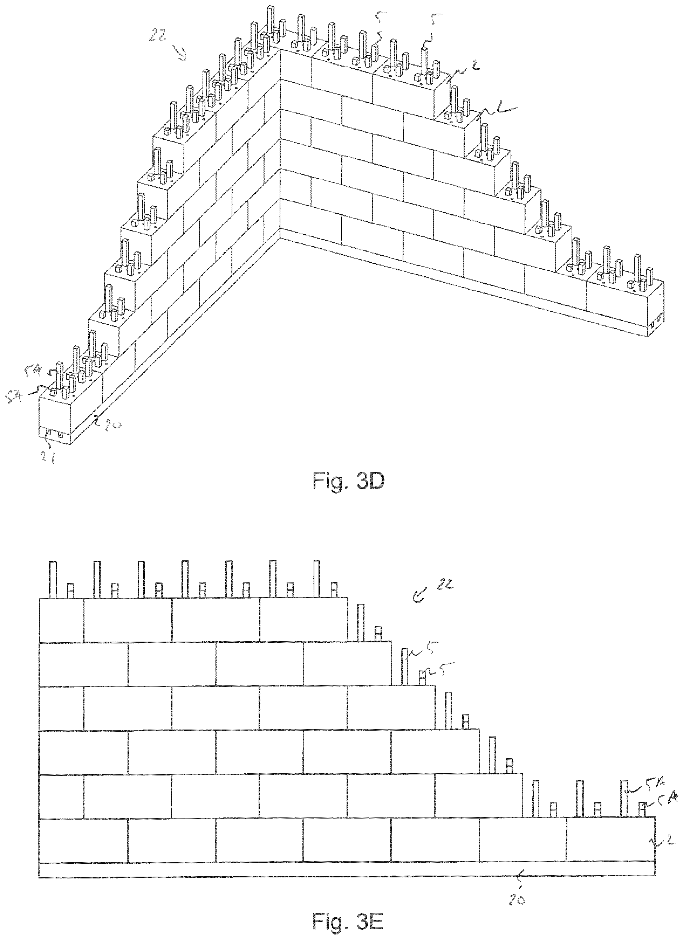

In a next step, shown in Figures 3D and 3E, second and further layers of construction element as shown in Figures 1A and 1B have been placed one on top of the other, with the construction elements in each subsequent layer staggered relative to the previous layer, to build a cornered wall 22. When a construction element is placed on top of a previous layer, the pegs in the previous layer push the pegs in the newly placed construction element up and out, generating an identical pattern of pegs sticking out the newly placed layer. This process repeats itself until the wall is finished. In the finished wall the pegs are aligned, essentially forming vertical, load-bearing beams inside the wall.

Figure 4 shows the cornered wall on a screw pile foundation, comprising further beams, e.g. steel profiles 25, secured in place by means of foundation screw piles 26 wound into the ground. The base 20 of the wall is placed on the beams 25.

Figure 5 shows a T-shaped wall 30, e.g. an external wall and an integrated internal wall, using a T-shaped block 2 with three groups of through-holes and pegs 5.

Figures 6 and 7 show a wall reinforced with tension anchors 35. The pegs 5 extending from the top layer have been sawn to equal lengths and a top plate 36 with grooves 37 has been placed over the top pegs 5 and blocks 2. The anchors extend through the base plate 20, the through-holes 7 (Figures 1a and 1B) and the top plate 36 and have been tensioned to press the plates and the construction elements between them together.

The blocks were made from a mycelium composite. In an example, the blocks were moulded by mixing a substrate, such as a blend of hemp, foliage, and sawdust, with a fungus (inoculum), for instance Pleurotus ostreatus, optionally at least one nutrient, such as oatflakes, and water.

After the mixture was introduced into a mould, the mould was covered, e.g. with an impermeable foil or tarpaulin, and the temperature of the mixture was maintained in a range from 15 to 24°C, for example 20°C. The fungus was allowed to grow, e.g. for a period in a range from 50 to 120 hours, preferably in a range from 70 to 110 hours, for example 100 hours, to form a network of hyphae through the mixture and into any additional wooden elements, such as such as battens, to form a mycelium composite.

When the mycelium composite was considered at or near optimum, in terms of strength, stiffness and durability in a dried state, the fungus was killed by heating and drying the prefab construction element and the mycelium composite.

The invention is not restricted to the above-described embodiments, which can be varied in a number of ways within the scope of the claims.

## Claims

1. Construction element, preferably a load-bearing construction element (1), for building a construction component, such as a wall (22, 30) or roof, and comprising a block (2) having opposite surfaces and one or more through-holes (3) extending from one surface to the other, **characterized in that** the block (2) is a molded block **and in that** at least one of the trough-holes (3) slidably holds a positioning and load-bearing peg (5).

2. Construction element (1) according to claim 1, wherein the block (2) comprises a plurality of through-holes (3) extending from one surface to the other and wherein at least two of the holes (3), preferably all of the holes (3), slidably hold a positioning and load-bearing peg (5).

3. Construction element (1) according to claim 1 or 2, wherein the peg or pegs (5) are clampingly held inside the through-holes (3).

4. Construction element (1) according to any one of the preceding claims, wherein the block (2) comprises tongues (10) and grooves (11) and/or comprises further through-holes (6, 7) or channels for conduits, electrical wires, and/or for tension anchors (35) and/or wherein one or more, preferably most, preferably all of the external edges of the block (2) are chamfered.

5. Construction element (1) according to any one of the preceding claims, wherein the through-holes (3) and the pegs (5) are cylindrical and/or wherein the block (2) is a parallelepiped.

6. Construction element (1) according to any one of the preceding claims, wherein the opposite surfaces have an aspect ratio of 1:N, where N is an integer.

7. Construction element (1) according to any one of the preceding claims, wherein the pattern of the through-holes (3), when seen in top view, comprises at least two groups of through-holes and wherein at least one of the groups is rotationally symmetrical, preferably of the order 4.

8. Construction element (1) according to any one of the preceding claims, wherein the distance between the side walls (12) of the block (2) and the pegs (5) is at least 5 cm preferably at least 7 cm.

9. Construction element (1) according to any one of the preceding claims, wherein the block (2) is made from mycelium, preferably from at least one fungus selected from the group consisting of Pleurotus ostreatus, Pleurotus eryngii, Stropharia Rugosoannulata, Trametes versicolor, Ganoderma Lucidum, Phanerochaete chrysosporium, Bjerkandera adusta, Lentinula edodes, Pycnoporus cinnabarinus, Pycnoporus sanguineus, Grifola frondosa, Schizophyllum commune, Neolentinus lepideus, and Heterobasidiom annosum.

10. Construction element (1) according to claim 9, wherein additional elements, in particular elements on the outer surface of the construction element, such as battens (15) providing fixing point for, in particular a façade or wall cladding, are integrated in the mycelium.

11. Construction element (1) according to any one of the preceding claims, wherein blocks are at least 50 centimeters (cm) long at least 20 cm wide, and at least 20 cm deep.

12. Kit for building a construction component (22, 30), comprising a plurality of construction elements (1) according to any one of the preceding claims, and a set of separate pegs (5A) and preferably a base (20) for the construction component (22, 30).

13. Kit according to claim 12, wherein the separate pegs (5A) have at least two, preferably at least three different lengths.

14. Method of building a construction component, such as a wall, roof or floor, comprising the steps of positioning a first layer of blocks (2) on a base (20) and positioning pegs (5A) in a pattern on the base (20), such that the pegs (5A) extend from the top surfaces of the blocks (2), and placing second and preferably further layers of construction elements (1) according to any one of the claims 1-9, on the first layer, such that the pegs (5A) in the first layer push the pegs (5) in the second layer of construction elements (1) up and out.

15. Method according to claim 14, wherein the pegs (5A) in the first series have different lengths.
